# EUROPÄISCHE PATENTSCHRIFT

(11) **EP 1 534 217 B1**
(45) Veröffentlichungstag und Bekanntmachung des Hinweises auf die Patenterteilung: **01.08.2007**
(21) Anmeldenummer: 03792317.4
(22) Anmeldetag: 14.08.2003
(51) Int. Cl.: A61Q 19/00, A61Q 17/00, A61K 8/29, A61K 8/73, A61K 8/81

(54) **DÜNNFLÜSSIGE KOSMETISCHE ODER DERMATOLOGISCHE ZUBEREITUNGEN VON TYP ÖL-IN-WASSER**
THIN LIQUID COSMETIC OR DERMATOLOGICAL PREPARATIONS OF THE OIL-IN-WATER TYPE
PREPARATIONS COSMETIQUES OU DERMATOLOGIQUES FLUIDES DU TYPE HUILE DANS L'EAU

(30) Priorität: 23.08.2002 DE 10238649
(43) Veröffentlichungstag der Anmeldung: 01.06.2005
(73) Patentinhaber: Beiersdorf AG, 20245 Hamburg (DE)
(72) Erfinder: STILLER, Sabine, 20251 Hamburg (DE); SCHULZ, Jens, 22869 Schenefeld (DE); DANIELS, Rolf, 38226 Salzgitter (DE); HAHN, Ingo, 22391 Hamburg (DE); VON DER FECHT, Stephanie, 22869 Schenenefeld (DE); GROTELÜSCHEN, Birgit, 22459 Hamburg (DE)
(86) Internationale Anmeldenummer: PCT/EP2003/009024
(87) Internationale Veröffentlichungsnummer: WO 2004/017930

(56) Entgegenhaltungen:
- EP-A- 0 667 144
- EP-A- 1 210 928
- EP-A- 1 352 639
- DE-A- 10 162 840

## Beschreibung

Die vorliegende Erfindung betrifft dünnflüssige, emulgatorfreie feindisperse kosmetische und dermatologische Zubereitungen vom Typ Öl-in-Wasser, insbesondere emulgatorfreie O/W-Emulsionen, welche eine Viskosität von weniger als 3000 mPa·s haben, sowie ihre Verwendung für kosmetische und medizinische Zwecke. Ferner betrifft die vorliegende Erfindung kosmetische und dermatologische Tücher, welche mit derartigen Zubereitungen impregniert sind.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze ÖI/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so können polare Ölkomponenten wie z.B. UV-Filter zu Instabilitäten führen. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar, welcher in den unterschiedlichsten Anwendungsgebieten eingesetzt wird. So stehen für die Pflege der Haut, insbesondere für die Rückfettung trockener Haut, eine Vielzahl von Produkten - wie Lotionen und Cremes - zur Verfügung. Ziel der Hautpflege ist es, den durch tägliches Waschen verursachten Fett- und Wasserverlust der Haut auszugleichen. Außerdem sollen Hautpflegeprodukte vor Umwelteinflüssen - insbesondere vor Sonne und Wind - schützen und die Hautalterung verzögern.

Kosmetische Emulsionen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Auch zur Reinigung der Haut und Hautanhangsgebilde finden Emulsionen in form von Reinigungsemulsionen Verwendung. Sie werden meist der Gesichtsreinigung und insbesondere zur Entfernung von dekorativer Kosmetik eingesetzt. Derartige Reinigungsemulsionen haben - im Gegensatz zu anderen Reinigungszubereitungen wie beispielsweise Seife - den Vorteil, besonders hautverträglich zu sein, da sie in ihrer lipophilen Phase pflegende Öle und/oder unpolare Wirkstoffe - wie z. B. Vitamin E - enthalten können.

Eine besondere Anwendungsform kosmetischer und/oder dermatologischer Emulsionen ist deren Verwendung als Tränkungslösung für kosmetische oder dermatologische Tücher.

Nachteilig am Stand der Technik ist die Tatsache, daß Emulsionen in der Regel organische Emulgatoren enthalten, welche die Emulsionen stabilisieren bzw. deren Herstellung erst ermöglichen. An sich ist die Verwendung von Emulgatoren in kosmetischen oder dermatologischen Zubereitungen zwar unbedenklich. Dennoch können Emulgatoren, wie letztlich jede chemische Substanz, im Einzelfall allergische oder auf Überempfindlichkeit des Anwenders beruhende Reaktionen hervorrufen. Als unerwünschte Nebenwirkungen können bei empfindlichen Personen beispielsweise Hautreizungen auftreten. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß bestimmte feinstverteilte Feststoffteilchen eine emulsionsstabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei insbesondere die Oberflächeneigenschaften der Feststoffpartikel.

Diese sogenannten Pickering-Emulsionen weisen in der Regel eine hohe Viskostät von mehr als 8.000 mPa·s auf. Fließfähige (mit einer Viskosität von etwa 3.000 mPa·s bis zu etwa 8.000 mPa·s) oder gar dünnflüssige (mit einer Viskosität von weniger als 3.000 mPa·s) O/W-Pickering-Emulsionen sind nach dem Stand der Technik nicht herstellbar.

Emulsionen mit einer sehr geringen Viskosität (dünnflüssige bzw. sprühbare Emulsionen) sind bislang - wenn überhaupt - nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Zwar beschreibt die Europäische Patentschrift 667 144 kosmetische Sonnenschutzmittel, welche Öl-in-Wasser-Emulsionen darstellen und anorganische Nanopigmente auf der Basis von Metalloxiden als Lichtschutzmittel enthalten, wobei die Formulierungen auch sprühbar sein können. Diese Zubereitungen stellen sogenannte PIT-Emulsionen dar, welche durch Phasenumkehr hergestellt werden und daher besonders feindispers sind. Allerdings konnte diese Schrift nicht den Weg zur vorliegenden Erfindung weisen.

Überhaupt haben dünnflüssige Zubereitungen des Standes der Technik häufig den Nachteil, daß sie auf einen engen Anwendungsbereich oder eine eingeschränkte Rohstoffauswahl begrenzt sind. Auch die Einarbeitung höherer Konzentrationen an polaren Ölkomponenten bereitet häufig Schwierigkeiten. Es ist aber gegebenfalls wünschenswert, hohe Mengen polarer Ölkomponenten in eine Formulierung einzuarbeiten, beispielsweise um feste UV-Filtersubstanzen lösen und so einen hohen Lichtschutzfaktor erreichen zu können.

Eine Aufgabe der vorliegenden Erfindung war es, emulgatorfreie Zubereitungen vom Typ Öl-in-Wasser herzustellen, welche eine sehr geringe Viskosität haben und nicht die Nachteile des Standes der Technik aufweisen. Eine weitere Aufgabe der Erfindung war, Lösungswege zu kosmetischen oder dermatologischen, möglichst dünnflüssigen O/W-Emulsionen aufzudecken, welche gegenüber erhöhten Elektrolytkonzentrationen stabil sind und in die sich hohe Mengen an polaren Ölkomponenten einarbeiten lassen. Ferner war Aufgabe der Erfindung, ein Verfahren zur Stabilisierung von dünnflüssigen O/W-Formulierungen zu finden.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen oder Deodorantien, aber auch ausgesprochen medizinisch-pharmazeutische Darreichungsformen geschaffen werden, zum Beispiel Zubereitungen gegen Akne und andere Hauterscheinungen. Ferner sollten auch Zubereitungen gefunden werden, welche sich als Tränkungslösungen für kosmetische oder dermatologische Tücher eignen.

Getränkte Tücher finden als Gegenstände des täglichen Bedarfs breiten Einsatz in unterschiedlichsten Bereichen. Sie erlauben unter anderem effiziente und hautschonende Reinigung und Pflege besonders auch in der Abwesenheit von (fließendem) Wasser. Dabei besteht der eigentliche Gebrauchsgegenstand aus zwei Komponenten:
a) einem trockenen Tuch, welches aus Materialien wie Papier und/oder unterschiedlichsten Mischungen aus Natur- oder Kunstfasern aufgebaut ist und
b) einer niederviskosen Tränkungslösung.

Kosmetische oder dermatologische Tücher können sowohl aus wasserlöslichen (z. B. wie Toilettenpapier) als auch aus wasserunlöslichen Materialien bestehen. Ferner können die Tücher glatt, gelocht oder auch oberflächenstrukturiert sein.

Herkömmliche Imprägnier-, bzw. Tränkungslösungen für wasserunlösliche Vliesmaterialien weisen bisher häufig den Mangel geringer Langzeitstabilität auf. Derartige Emulsionen neigen insbesondere bei erhöhter Umgebungstemperatur zur Phasentrennung, was einen entscheidenden Nachteil für den Imprägnierungsprozeß sowie für die finale Qualität des Endproduktes darstellt.

Die Langzeitstabilität von Imprägnier- bzw. Tränkungslösungen des Standes der Technik wird im allgemeinen durch den Einsatz erhöhter Emulgatorkonzentrationen sowie starken Energieeintrag - beispielsweise bei der Homogenisation - gewährleistet.

Aufgabe der vorliegenden Erfindung war es daher, langzeitstabile Imprägnier - bzw. Tränkungslösungen zum Aufbringen auf wasserunlösliche Vliesmaterialien zu finden, die die Nachteile des Standes der Technik nicht zeigen und die bereits bei sehr geringen Emulgatorgehalten bzw. in Form von emulgatorfreien Formulierungen dünnflüssige, langzeitstabile O/W-Emulsionen darstellen, welche möglichst nicht homogenisiert werden müssen.

Es war erstaunlich und für den Fachmann in keiner Weise vorauszusehen, daß dünnflüssige Pickering-Emulsionen, welche feindisperse Systeme vom Typ Öl-in-Wasser darstellen und eine Viskosität von weniger als 3.000 mPa·s aufweisen, enthaltend
(1) eine Ölphase,
(2) eine Wasserphase,
(3) mindestens einen Typ mikrofeiner, anorganischer Partikel gewählt aus der Gruppe der Metalloxide, die
   a) eine mittlere Partikelgröße von weniger als 200 nm haben,
   b) sowohl hydrophile als auch lipophile Eigenschaften zeigen,
   c) sowohl in hydrophilen als auch in lipophilen Medien dispergierbar sind und
   d) gegebenenfalls oberflächlich beschichtet sind,
(4) 0,01 bis 5 Gew.-% mindestens eines polymeren nichtionischen und/oder kationischen Verdickungsmittels gewählt aus der gruppe Hydroxyethylcellulose und Polyquaternium 37 und
(5) weniger als 0,5 Gew.-% eines oder mehrerer Emulgatoren
den Nachteilen des Standes der Technik abhelfen.

Es ist erfindungsgemäß besonders vorteilhaft, wenn die Zubereitungen deutlich weniger als 0,5 Gew.-% eines oder mehrerer Emulgatoren enthalten. Ganz besonders bevorzugt sind erfindungsgemäße. Zubereitungen, welche gänzlich frei von Emulgatoren im herkömmlichen Sinn sind.

Es ist erfindungsgemäß ferner besonders vorteilhaft, wenn die Zubereitungen eine Viskosität von weniger als 2.000 mPa·s aufweisen.

Die erfindungsgemäßen Zubereitungen sind in jeglicher Hinsicht überaus befriedigende Präparate, die gegenüber herkömmlichen Pickering-Emulsionen eine deutlich geringere Viskosität haben und daher insbesondere geeignet sind, um als Grundlage für Zubereitungsformen mit vielfältigen Anwendungszwecken zu dienen. Es war insbesondere erstaunlich, daß durch die Kombination von erfindungsgemäßen mikrofeinen, anorganischen Partikeln mit nichtionischen und/oder kationischen Verdickungsmitteln dünnflüssige Zubereitungen zugänglich sind, wohingegen bei Verwendung von anionischen Verdickungsmitteln die Emulsionsstruktur zerstört wird.

Die erfindungsgemäßen Zubereitungen stellen in jeder Hinsicht eine Bereicherung des Standes der Technik in bezug auf dünnflüssige O/W-Emulsionen dar.

Pickering-Emulsionen des Standes der Technik (im folgenden auch Primäremulsionen genannt) sind durch Dispergieren von für die Herstellung von Pickering-Emulsionen geeigneten amphiphilen Partikeln in einer der beiden Emulsionphasen erhältlich. Die Phasenvereinigung erfolgt vorteilhaft unter Homogenisation mittels Beco-Mischer oder anderen geeigneten Homogenisationsanlagen. Die Homogenisation kann sowohl kalt (d. h. bei weniger als 30 °C) oder heiß (d. h. bei mehr als 60 °C) erfolgen.

Es war überraschend und nicht vorhersehbar, daß erfindungsgemäße fließfähige und dünnflüssigen Zubereitungen durch nachträgliches Verdünnen von verdickungsmittelfreien Pickering-Emulsionen des Standes der Technik erhältlich sind, d. h. durch Verdünnen von hochviskosen Primäremulsionen, welche keine Verdickungsmittel enthalten. Dazu werden diese mit einer bis zu zwanzigfachen Menge einer Verdünnungslösung vermischt, welche in einer wäßrigen Grundlage 0,01 bis 5 Gew.-% eines oder mehrerer erfindungsgemäßer nichtionischer und/oder kationischer Verdickungsmittel enthält.

### Gegenstand der Erfindung ist daher auch ein

Verfahren zur Herstellung von O/W-Pickering-Emulsionen mit einer Viskosität von weniger als 3.000 mPas, dadurch gekennzeichnet, daß eine O/W-Primäremulsion, welche .
(1) eine Ölphase,
(2) eine Wasserphase,
(3) mindestens einen Typ mikrofeiner, anorganischer Partikel gewählt aus der Gruppe der Metalloxide, die
   a) eine mittlere Partikelgröße von weniger als 200 nm haben,
   b) sowohl hydrophile als auch lipophile Eigenschaften zeigen,
   c) sowohl in hydrophilen als auch in lipophilen Medien dispergierbar sind, enthält und welche eine Viskosität von 8.000 mPa·s oder mehr hat, mit einer bis zu zwanzigfachen Menge einer wässrigen Lösung, in der ein geeignetes Verdickungmittel gleichmäßig verteilt vorliegt, vermischt wird, wobei die O/W-Pickering-Emulsionen weniger als 0,5 Gew.-% eines oder mehrerer Emulgatoren enthalten.

Es ist auch möglich im Sinne der vorliegenden Erfindung, das wässrige Verdünnungsmedium (ohne Verdickungsmittelzusatz) und das Verdickungsmittel separat zur Pickering-Primäremulsion zuzugeben, sofern eine gleichmäßige Durchmischung - beispielsweise durch eines der im folgenden genannten Verfahren - erreicht werden kann.

Die Vermischung kann vorteilhaft durch. Rühren (z. B. mittels Blattrührer) bei einer mittleren Rührgeschwindigkeit von mehr als 50 U/min oder aber auch durch kräftiges Schütteln in einem geeigneten Gefäß, welches zur besseren Durchmischung vorteilhaft auch Mischungselemente wie z. B. Kunststoffkugeln enthalten kann, erfolgen.

Die Verdünnung der Primäremulsion im Sinne der vorliegenden Erfindung kann unabhängig vom Zeitpunkt ihrer Herstellung erfolgen.

Polymere Verdickungsmittel im Sinne der vorliegenden Erfindung sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche, in Wasser dispergierbare natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken.

Erfindungsgemäß vorteilhaft sind beispielsweise die im folgenden aufgelisteten nichtionischen und kationischen polymeren Verdickungsmittel:
- organische, abgewandelte Naturstoffe, wie z. B. Hydroxyethylcellulose und andere Celluloseether, Hydroxyethyl- und - propylcellulose und dergleichen, sowie modifizierte Stärkederivate
- organische, natürliche Verbindungen, wie beispielsweise Agar-Agar, Carrageen, Polyosen, Stärke, Dextrine, Gelatine, Casein,
- organische, vollsynthetische Verbindungen, wie z. B. Vinylpolymere, Polyether, Polyimine, Polyamide und Derivate der Polyacrylsäure,
- anorganische Verbindungen, wie z. B. Polykieselsäuren, Tonmineralien wie Montmorillonit, Zeolithe, Kieselsäuren.

Vorteilhaft im Sinne der vorliegenden Erfindung sind beispielsweise Alkylcellulosen, als welche die Alkylether der Cellulose bezeichnet werden. Sie zeichnen sich durch die folgende Strukturformel aus 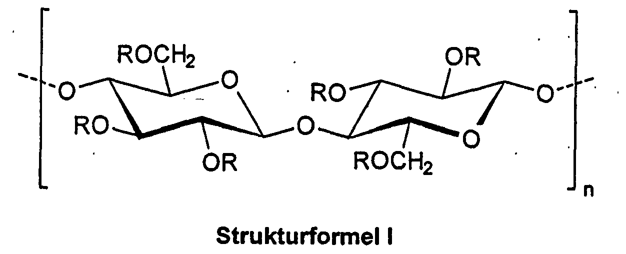 in der R ein Wasserstoff oder eine Alkylgruppe mit 1 bis 4 Kohlenstoffatomen darstellen kann.

Erfindungsgemäß bevorzugte Alkylcellulose ist Hydroxyethylcellulose (CAS 9004-62-0), beispielsweise die unter der Handelsbezeichnung Natrosol Hydroxyethylcellulose bei der Fa. Aqualon erhältliche.

Insbesondere vorteilhaft im Sinne der vorliegenden Erfindung sind ferner Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen:

Vorteilhafte kationische Verdickungsmittel im Sinne der vorliegenden Erfindung sind ferner kationisch modifizierte Polyacrylsäurederivate, insbesondere solche, die aus Homopolymeren aus Methacryloylethyltrimethylammonium Chlorid bestehen (INCI: Polyquaternium 37), beispielsweise erhältlich unter der Bezeichnung Synthalen CR bei der FA 3V Sigma.

Die Gesamtmenge an einem oder mehreren polymeren Verdickungsmitteln wird in den fertigen kosmetischen oder dermatologischen O/W-Pickering-Emulsionen vorteilhaft kleiner als 5,0 Gew.-%, bevorzugt zwischen 0,05 und 3 Gew.-%, jeweils bezogen auf das Gesamtgewicht der Zubereitungen gewählt.

Es ist ferner vorteilhaft, wenngleich nicht zwingend, wenn die erfindungsgemäßen Pikkering-Emulsionen weitere Hilfsstoffe enthalten, die die Stabilität dieser Zubereitungen zusätzlich erhöhen können, beispielsweise Stoffe, die gewählt werden aus der Gruppe der Wachse und/oder Ölverdickungsmittel sowie der Elektrolyte.

Es ist ferner vorteilhaft, wenn die erfindungsgemäßen Pickering-Emulsionen Hilfsstoffe enthalten, die zur Verminderung oder Verhinderung eines stumpfen oder trockenen Hautgefühls nach deren Auftragen beitragen können, wobei der hauptsächliche Zweck dieser Stoffe ein anderer sein kann. Vorzugsweise werden diese Stoffe beispielsweise gewählt aus der Gruppe der unsymmetrisch substituierten s-Triazinderivate, der Cyclodextrine, der Filmbildner und der polymeren Moisturizer, wobei diese Stoffe sowohl einzeln als auch im Gemisch vorliegen können.

Die kosmetischen Eigenschaften der erfindungsgemäßen Pickering-Emulsionen können zusätzlich beispielsweise noch dadurch verbessert werden, daß in der Ölphase auch Öle eingesetzt werden, welche eine Viskosität haben, die kleiner als 30 mPa·s, insbesondere kleiner als 20 mPa·s ist (bestimmt mit einem Rheometer der Firma Contraves (Rheomat 108E) bei einem Schergefälle von 500/s und einer Temperatur von 25 °C).

Die kosmetischen Eigenschaften (insbesondere die Benetzungseigenschaften und das Hautgefühl) der erfindungsgemäßen Pickering-Emulsionen können zusätzlich ferner noch durch den Einsatz von Chitosan (in Konzentrationen kleiner 1 Gew.-%, insbesondere 0,1 bis 0,5 Gew.-%) und/oder anderen kationischen Filmbildnern, wie z. B. Stärke Hydroxypropyltrimonium Chlorid, welches unter der Handelsbezeichnung Sensomer Cl-50 von der FA Ondeo Nalco erhältlich ist, verbessert werden.

Es ist ferner vorteilhaft, wenn die erfindungsgemäßen Pickering-Emulsionen Lösungsvermittler enthalten; insbesondere vorteilhaft sind PPG-15 Stearyl Ether und PEG-40 Hydrogenated Castor Oil.

### Mikrofeine anorganische Partikel:

Der amphiphile Charakter der erfindungsgemäßen mikrofeinen Partikel zeigt sich beispielsweise darin, daß diese sowohl in hydrophilen als auch in lipophilen Flüssigkeiten dispergierbar sind. Dementsprechend können auch Pigmente, die vom Hersteller als hydrophob charakterisiert werden, stabilisierende Eigenschaften haben.

Um eine nähere Qualifizierung der Oberflächeneigenschaften zu ermöglichen, eignen sich Benetzbarkeitsbestimmungen. Dabei wird das Sedimentationsverhalten der Pigmente in unterschiedlich konzentrierten Ethanol-Wasser-Mischungen beobachtet, um die Stabilisierungseigenschaften von Pigmenten einschätzbar zu machen. Dazu wird diejenige Zeit gemessen, die ein Pigment benötigt, um vollständig in eine Flüssigkeit einzutauchen. Als benetzbar gelten Pigmente, die innerhalb von 10 min vollständig in eine Flüssigkeit eingetaucht sind. Zur Stabilisierung von Pickering-Emulsionen sind Pigmente geeignet, die durch eine 40 %ige Ethanol-Wasser-Mischung benetzbar sind.

Es ist vorteilhaft, den mittleren Partikeldurchmesser der verwendeten Partikel zwischen 1 nm und 200 nm, besonders vorteilhaft zwischen 5 nm und 100 nm zu wählen.

Es ist ferner vorteilhaft, die Konzentration aller erfindungsgemäßen amphiphilen Partikel größer als 0,1 Gew.-%, vorteilhaft zwischen 0,1 Gew.-% und 30 Gew.-%, besonders vorteilhaft zwischen 1 Gew.-% und 15 Gew.%, jeweils bezogen auf das Gesamtgewicht der Zubereitungen, zu wählen. -

Vorteilhaft im Sinne der vorliegenden Erfindung sind alle Partikel, die geeignet sind, dünnflüssige Pickering-O/W-Emulsionen zu stabilisieren. Im wesentlichen unerheblich für die vorliegende Erfindung ist es, in welcher der gegebenenfalls natürlich vorkommenden Modifikationen die Partikel vorliegen.

Vorzugsweise werden zur Stabilisierung der Pickering-Emulsionen unbehandelte, nahezu reine Pigmentpartikel verwendet, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck und Tayca erhältlichen Titandioxid-Pigmente sowie bei der Firma Tayca und BASF erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden ebenfalls vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | Dimethicone | BASF |
| Z- Cote | / | BASF |
| ZnO NDM | Dimethicone | H&R |
| NanoX | / | Harcros Chemical |
| Zinkoxid neutral | / | H&R |
| MZ- 707S | Methicone | Tayca Corporation |
| MZ- 707M | Dimethicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| Eusolex T-2000 | Alumina / Simethicone | Merck KgaA |
| MT-100TV | Alumina / Stearinsäure | Tayca Corporation |
| MT-100Z | Alumina / Stearinsäure | Tayca Corporation |
| UV-Titan X610 | Alumina / Dimethicone | Kemira |
| MT-150A | / | Tayca Corporation |
| MT-500B | / | Tayca Corporation |
| MT-100SA | Alumina / Silica | Tayca Corporation |
| MT-01 | Alumina / Stearinsäure | Tayca Corporation |
| Titandioxid T805 (Uvinul TiO₂) | Octyltrimethylsilan | Degussa |
| Eusolex T-aqua | Wasser / Alumina / Natriummetaphosphat | Merck |

Ganz besonders vorteilhaft im Sinne der vorliegenden Erfindung sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Es ist ferner vorteilhaft, wenngleich nicht zwingend, die erfindungsgemäßen mikrofeinen Partikel mit weiteren amphiphilen Partikeln zu kombinieren, welche gegebenenfalls auch zur Stabilisierung der Pickering-Emulsionen beitragen können.

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkemöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethyl-hexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an. C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hatistar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen. '

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen -Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 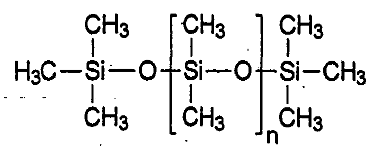 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Zubereitungen gemäß der vorliegenden Erfindung können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern :
(a) Siloxanelastomere, welche die Einheiten R₂SiO und RSiO_{1,5} und/oder R₃SiO_{0.5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, daß die Menge des Wasserstoffes des Organopolysiloxans (1) oder der ungesättigten aliphatischen Gruppen des Organopolysiloxans (2)
- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel , ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem Rowe Colour Index, 3. Auflage, Society of Dyers and Colourists, Bradford, England, 1971 zu wählen.

Sofern die erfindungsgemäßen Formulierungen in Form von Produkten vorliegen, welche im Gesicht angewendet werden, ist es günstig, als Farbstoff eine oder mehrere. Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthyl-azo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | TiO₂: 40 - 60 nm | silber |
| Interfererizpigmente | TiO₂ 60 - 80 nm | gelb |
| | TiO₂ 80 -100 nm | rot |
| | TiO₂: 100 -140 nm | blau |
| | TiO₂: 120 -160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ Goldtöne | Goldtöne |
| | TiO₂ / Cr₂O₃ | grün |
| | TiO₂ / Berliner Blau | tiefblau |
| | TiO₂ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im Allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Die erfindungsgemäßen Pickering-Emulsionen können als Grundlage für kosmetische oder dermatologische Formulierungen dienen. Diese können wie üblich zusammengesetzt sein und beispielsweise zur Behandlung und der Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als-Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel,' anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist. Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Insbesondere können die erfindungsgemäßen Pickering-Emulsionen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. a-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B: Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und. Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid). Es gibt laut Dr. Schönrock Vorbeschreibungen zur Thematik Titandioxid in Kombination mit Dioic acid von der FA Unilever. Vielleicht sollten wir da nochmal Rücksprache halten?Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren und Elektrolyte.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan, Starch Hydroxypropyltrimonium Chloride und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können, vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende, Wirkung haben (wie z. B. Bornitrid etc.) und/oder Aerosile^{®} (CAS-Nr. 7631-86-9).

Vorteilhaft im Sinne der vorliegenden Erfindung sind auch Zubereitungen, die in Form von Reinigungsemulsionen vorliegen, welche vorteilhaft zwar keine waschaktiven Tenside enthalten, aber beispielsweise zum Entfernen von Schminken und/oder Make-up oder als milder Waschschaum - ggf. auch für unreine Haut - verwendet werden können. Derartige Reinigungsemulsionen können vorteilhaft ferner als sogenannte "rinse off" Präparate angewendet werden, welche nach der Anwendung von der Haut abgespült werden.

Günstig sind auch kosmetische und dermatologische Zubereitungen, die in der Form eines Sonnenschutzmittels vorliegen. Es ist aber auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der. Wasser- und/oder der Ölphase vorliegen können.

Die Zubereitungen gemäß der vorliegenden Erfindung können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylecrylat (INCI: Octocrylene), 2-Ethylhexyl-2-hydroxybenzoat (2-Ethyl-hexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃ Zirkoniums (ZrO₂), Siliciums (SiO₂), Mangans (z.B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und StyrollAcrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert;-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 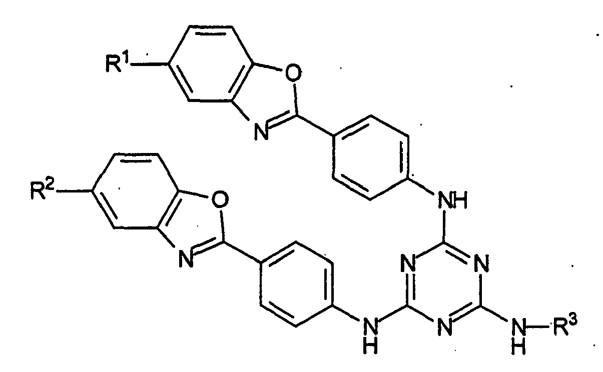 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 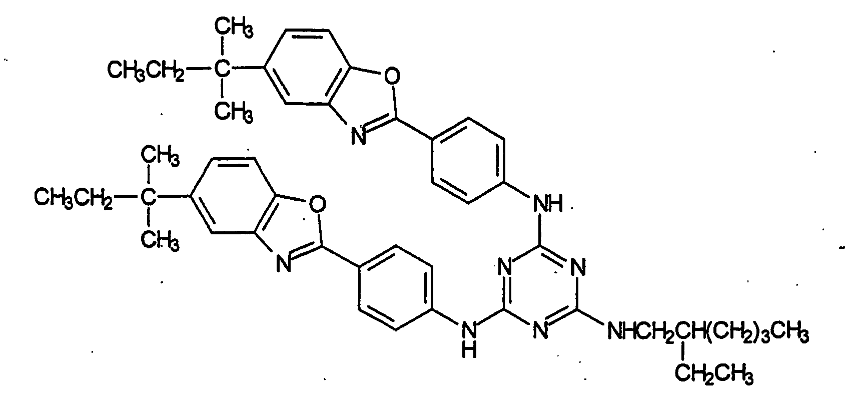 auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 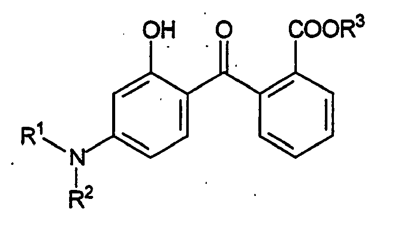 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 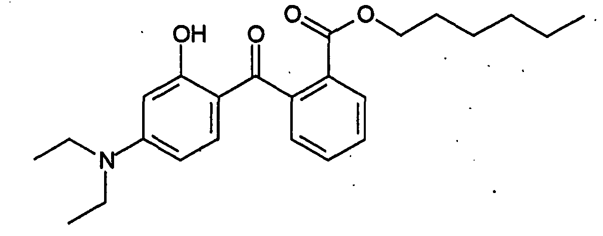 und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.
Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die Liste der genannten UV-Filter, die im Sinne der vorliegenden Erfindung eingesetzt werden können, soll selbstverständlich nicht limitierend sein.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Desodorantien betrifft.

In sogenannten Antitranspirantien kann durch Adstringentien - vorwiegend Aluminiumsalze wie Aluminiumhydroxychlorid (Aluchlorhydrat) - die Bildung des Schweißes reduziert werden. Ferner ist die Kombination von Adstringentien mit antimikrobiell wirksamen Stoffen in ein und derselben Zusammensetzung gebräuchlich. Auch Antitranspirantien sind vorteilhaft im Sinne der vorliegenden Erfindung.

Die Liste der genannten Wirkstoffe bzw. Wirkstoffkombinationen soll selbstverständlich nicht limitierend sein.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Erfindungsgemäß bevorzugt werden die erfindungsgemäßen Pickering-Emulsionen als Tränkungslösungen in Kombination mit "trockenen" Tüchern eingesetzt. Die Tücher können aus wasserlöslichen, vorteilhaft aber aus wasserunlöslichen Materialien bestehen. Ferner können die Tücher im Sinne der vorliegenden Erfindung glatt, gelocht oder oberflächenstrukturiert sein. Besonders vorteilhaft im Sinne der vorliegenden Erfindung sind Tücher, welche aus Vlies bestehen, insbesondere aus wasserstrahlverfestigten und/oder wasserstrahlgeprägten Vlies.

Derartige Vliese können Makroprägungen jeden gewünschten Musters aufweisen. Die zu treffende Auswahl richtet sich zum einen nach der aufzubringenden Tränkung und zum anderen nach dem Einsatzfeld, auf dem das spätere Tuch Verwendung finden soll.

Werden geprägte Vliese verwendet, so erleichtern große Kavitäten an der Vliesoberfläche und im Vlies die Aufnahme von Schmutz und Verunreinigungen, wenn mit dem getränkten Tuch über die Haut gefahren wird. Die Reinigungswirkung kann gegenüber ungeprägten Tüchern um ein Vielfaches gesteigert werden.

Es hat sich als vorteilhaft herausgestellt für das Tuch, wenn dieses ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Die Dicke des Vlieses beträgt vorzugsweise 0,4 mm bis 2 mm, insbesondere 0,6 mm bis 0,9 mm.

Als Ausgangsmaterialien für den Vliesstoff des Tuches können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 Gew.-% Viskose und 30 Gew.-% PET. Ferner bevorzugt sind Vliese aus PET, Viskose und Baumwolle, beispielsweise solche aus 40 Gew.-% PET, 50 Gew.-% und 10 Gew.-% Baumwolle oder solche aus 50 Gew.-% PET, 40 Gew.-% und 10 Gew.-% Baumwolle.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Tuches eingesetzten Fasern weisen vorzugsweise eine Wasseraufnahmerate von mehr als 60 mm/[10 min] (gemessen mit dem EDANA Test 10.1-72), insbesondere mehr als 80 mm/[10 min] auf.

Fernen weisen die zur Bildung des Tuches eingesetzten Fasern vorzugsweise ein Wasseraufnahmevermögen von mehr als 5 g/g (gemessen mit dem EDANA Test 10.1-72), insbesondere mehr als 8 g/g auf.

Vorteilhafte Tücher im Sinne der vorliegenden Erfindung haben eine Reißkraft von insbesondere

| | | [N/50mm] |
|---|---|---|
| im trockenen Zustand | Maschinenrichtung | >60, vorzugsweise >80 |
| | Querrichtung | >20, vorzugsweise >30 |
| im getränkten Zustand | Maschinenrichtung | >4, vorzugsweise >60 |
| | Querrichtung | >10, vorzugsweise >20 |

Die Dehnfähigkeit vorteilhafter Tuches beträgt vorzugsweise

| | | |
|---|---|---|
| im trockenen Zustand | Maschinenrichtung | 15 % bis 100 %, bevorzugt 20 % und 50% |
| | Querrichtung | 40 % bis 120 %, bevorzugt |
| | | 50 % und 85 % |
| im getränkten Zustand | Maschinenrichtung | 15 % bis 100 %, bevorzugt 20 % und 40 % |
| | Querrichtung | 40 % bis 120 %, bevorzugt 50 % und 85 % |

Vorteilhafte erfindungsgemäße Tränkungslösungen haben eine Viskosität von weniger als 2000 mPa·s. Derartige Tränkungslösungen lassen sich in an sich bekannter Weise auf das Tuch aufgetragen.

Erfindungsgemäß vorteilhaft sind nicht nur feuchtgetränkte Tücher, sondern auch sogenannte "trockene Tücher" (engl. "Dry Wipes"), welche beispielsweise erhältlich sind durch abschließendes Trocknen von feuchtgetränkten Tüchern. Diese Tücher können vom Anwender durch einfaches Befeuchten wieder anwendungsbereit gemacht werden.

Erfindungsgemäß ist auch die Verwendung der erfindungsgemäßen Tücher zur Reinigung und Pflege der Haut sowie die Verwendung von erfindungsgemäßen Tüchern als Gesichtstücher, Babytücher, Intimpflegetücher, Deotücher, Sonnenschutztücher und/oder Tücher zur Pflege und/oder Reinigung sensibler Haut.

Besonders vorteilhaft an den erfindungsgemäßen dünnflüssigen Emulsionen ist, daß sich mit ihrer Hilfe Pigmente, wie z. B. ZnO, auf ein Tuch aufbringen lassen. Das Aufbringen von Zinkoxid ist insbesondere dann bevorzugt, wenn die erfindungsgemäßen Tücher in der Babypflege eingesetzt werden sollen. Durch das Aufbringen von ZnO, welches für seine antibakteriellen Eigenschaften bekannt ist, wird bei der täglichen Reinigung ein zusätzlicher Schutz vor Windeldermatitis erzielt.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiel 1:

### Die Primäremulsion 1 mit einer Viskosität >8000mPa bestehend aus

| | |
|---|---|
| **Primäremulsion** | **1** |
| Titandioxid (Eusolex T2000) | 2,5 |
| Zinkoxid | 4 |
| Di Stärkephosphat | 1,5 |
| Hydrogenierte Coco Glyceride | 1 |
| C₁₆₋₃₈ Alkylhydroxystearoyl-stearat (Kesterwachs K80P) | 0,5 |
| Cetyl Dimethicon (Abil Wax 9840) | 0,5 |
| Octyldodecanol | 3,75 |
| Butylen Glycol Caprylat/Caprat | 5 |
| PVP/ Hecadecene Copolymer | 0,5 |
| Stearyl Alcohol | 1,5 |
| Cyclomethicon | 2 |
| Tocopheryl Acetat | 0,5 |
| PPG-15 Stearyl Ether | 1 |
| Dicaprylyl Ether (Cetiol OE) | 3,5 |
| Ethylhexyl Methoxycinnamat | 5 |
| Bis- Ethylhexyloxyphenol Methoxyphenyl Triazin | 2 |
| Octocrylene | 3,5 |
| Aminobenzophenon | 2,5 |
| Ethylhexyltriazon | 2 |
| Dronetrizole Trisiloxan | 1,5 |
| Phenyl Dibenzimidazol Tetrasulfonsäure | 2 |
| Konservierung | 1 |
| Glycerin | 5 |
| NaOH 45%ige Lösung in Wasser | 0;5 |
| EDTA-Lösung | 1 |
| Parfum | 0,4 |
| Wasser | ad 70 |

wird mit Verdünnungslösung 1, bestehend aus:

| | |
|---|---|
| **Verdünnungslösung** | **1** |
| Hydroxyethylcellulose | 1 |
| Polyquatemium 37 | 0,1 |
| Wasser | 200 |

unter 5-minütigem Rühren mittels Blattrührer bei einer Rührgeschwindigkeit von 100U/min vermischt.

### Beispiel 2:

Die Primäremulsion 2 mit einer Viskosität >8000mPa bestehend aus:

| | |
|---|---|
| **Primäremulsion** | **2** |
| Titandioxid (Eusolex T2000) | 2 |
| Talkum (Talkum Micron) | 1 |
| Di Stärkephosphat | 1 |
| Tapiokastärke | 3 |
| C₂₀₋₄₀ Alkylstearat (Kesterwachs K82) | 0,5 |
| Behenoxy Dimethicon (Abil Wax 2440) | 1 |
| Polyisobuten (Rewopal PIB 1000) | 0,5 |
| Caprylic/Capric Triglycerid | 5 |
| Octyldodecanol | 5 |
| C₁₂₋₁₅ Alkyl Benzoat | 5 |
| Acetylated Glycol Stearate + Tristearin | 2 |
| Stearyl Alcohol | 1 |
| Dimethicon | 2 |
| Tocopheryl Acetat | 1 |
| Starch Hydroxypropyltrimonium Chlorid (Sensomer Cl 50) | 1 |
| Ethylhexyl Methoxycinnamat | 5 |
| Bis- Ethylhexyloxyphenol Methoxyphenyl Triazin | 1,5 |
| Methylene Bis-Benzotriazolyl | 1 |
| Tetramethylbutylphenol | |
| Octocrylene | 3 |
| Terphthaliden Dicamphor Sulfonsäure | 0,5 |
| Dronetrizole Trisiloxan | 1 |
| Dihydroxyaceton | 2,5 |
| Benzoxazol | 0,5 |
| Glycerin | 5 |
| Biosaccharid Gel (Fucogel 1000) | 0,5 |
| Hyaluronsäure | 0,5 |
| EDTA-Lösung | 1 |
| wasserlöslicher Farbstoff | 0,3 |
| Alkohol | 15 |
| Parfum | 0,3 |
| Wasser | ad 70 |

wird mit Verdünnungslösung 2, bestehend aus:

| | |
|---|---|
| **Verdünnungslösung** | **2** |
| Hydroxypropyl Methylcellulose | 2 |
| Polyquaternium 37 | 1 |
| Wasser | 300 |

unter 2-minütigem, kräftigen Schütteln vermischt.

### Beispiel 3:

### Die Primäremulsion 3 mit einer Viskosität >8000mPa bestehend aus:

| | |
|---|---|
| **Primäremulsion** | **3** |
| Titandioxid (Eusolex T2000) | 5 |
| Zinkoxid | 5 |
| Silica (Aerosil R972) | 5 |
| Konservierung | 10 |
| Glycerin | 10 |
| Panthenol | 1 |
| Primäremulsion | 3 |
| Citronensäure | 8 |
| Natrium Citrat | 2 |
| Kalium Sorbat | 1 |
| EDTA-Lösung | 6 |
| fettlöslicher Farbstoff | 2 |
| Parfum | 5 |
| Wasser | ad 70 |

wird mit Verdünnungslösung 3, bestehend aus:

| | |
|---|---|
| **Verdünnungslösung** | **3** |
| Hydroxyethylcellulose | 6 |
| Polyvinylpyrrolidon | 2 |
| Wasser | ad 930 |

unter 5-minütigem Rühren mittels Blattrührer bei einer Rührgeschwindigkeit von 100U/min vermischt.

Beispiele für erfindungsgemäße direkt anwendbare dünnflüssige Pickeringemulsionen sind:

| | **4** | **5** | **6** | **7** | **8** |
|---|---|---|---|---|---|
| Titandioxid (Eusolex T2000) | 3 | 2 | 3 | 2 | 0,35 |
| Zinkoxid | | 4 | | | 0.65 |
| Titandioxid (Titandioxid T805) | 3 | | | 3 | |
| Silica (Aerosil R972) | | | | 0,5 | |
| Talkum (Talkum Micron) | 2 | | | 2 | 0,5 |
| Bornitrid | | | | 1 | |
| Natrium Maisstärke n-Octenylsuccinat | | 1 | 0,5 | | |
| Di Stärkephosphat | | 1 | | | |
| Tapiokastärke | 4 | 5 | | | |
| Hydrogenierte Coco Glyceride | 1 | 1 | | | |
| C₁₆₋₃₈ Alkylhydroxystearoyl-stearat (Kesterwachs K80P) | | | 3 | | |
| Behenoxy Dimethicon (Abil Wax 2440) | | 1 | | | |
| Cetyl Dimethicon (Abil Wax 9840) | | | | 1 | |
| Caprylic/Capric Triglycerid | | 4 | 8 | 5 | |
| Octyldodecanol | 4,25 | 4 | 8 | | |
| Mineralöl | 3,65 | 4 | 8 | | ' 8 |
| Butylen Glycol Caprylat/Caprat | 5 | | | 5 | |
| C₁₂₋₁₅ Alkyl Benzoat | | | 5 | 3 | |
| PVP/ Hecadecene Copolymer | 0,5 | | | 1 | |
| Acetylated Glycol Stearate + Tristearin | | | | 0,5 | |
| Stearyl Alcohol | 1,5 | 1 | | | |
| Dimethicon | | | | 2 | |
| Cyclomethicon . | 2 | 3 | | | |
| Tocopheryl Acetat | 0,5 | | | 1 | |
| Ubiquinon | | 0,01 | | | |
| PEG-40 Hydrogenated Castor Oil | 1 | | | | |
| Dicaprylyl Ether (Cetiol OE) | | | 5 | 3 | |
| Hydriertes Polyisobuten (Polysynlan) | | | | 0,1 | |
| Ethylhexyl Methoxycinnamat | 5 | | | 4 | |
| Bis- Ethylhexyloxyphenol Methoxyphenyl Triazin | 1,7 | | | 1 | |
| Methylene Bis-Benzotriazolyl Tetramethylbutylphenol | | | | 1 | |
| Octocrylene | , 3,5 | | | 2 | |
| Aminobenzophenon | | | | 0,5 | |
| Diethylhexyl Naphthalate | | | 4 | | |
| Ethylhexyltriazon | | | 4 | | |
| Terphthaliden Dicamphor Sulfonsäure | | | 0,5 | | |
| Butyl Methoxydibenzoylmethan | | | 2 | 2 | |
| Dihydroxyaceton | | 5 | | | |
| Diethylhexyl Butamido Triazon (UVASORB HEB) | | | | 1 | |
| Benzoxazol | 1 | | | | |
| Phenyl Dibenzimidazol Tetrasulfonsäure | | | 2 | 2 | |
| Phenylbenzimidazol Sulfonsäure | | | | 2 | |
| Konservierung | 1 | 1 | | | 0,6 |
| Glycerin | | 7 3 | 5 | | 3 |
| Aluminiumchlorohydrat | | | | | 10 |
| Panthenol | | 1 | | | |
| Hyaluronsäure | | | | 0,5 | |
| Chitosan | 0,5 | | | | 0,25 |
| Milchsäure | 0,3 | 0.8 | | | 0,3 |
| Citronensäure | | | | | 0,09 |
| Natrium Citrat | | | | | 0,18 |
| Kalium Sorbat | | | | | 0,05 |
| NaCl | | | 0,9 | | |
| Carbomer (Carbopol 981) | | | 0,1 | | |
| C₁₀₋₃₀ Alkyl Acrylat Crosspolymer (Pemulen TR1) | | | 0,3 | | |
| Dimethicon / Polysilicone-11 | | | | 0,2 | |
| Xanthan Gummi | | | 0,1 | | |
| Hydroxyethylcellulose | 0,4 | | . | | 0,4 |
| Hydroxypropyl Methylcellulose | | 0,1 | | | 0,2 |
| Methylhydroxyethylcellulose | 0,1 | | | 0,8 | 0,1 |
| Polyquaternium 37 | | 2 | | | |
| Hydroxypropyl Stärke Phosphat | | | 3 | | |
| Polyacrylat | | | | 0,1 | |
| NaOH 45%ige Lösung in Wasser | | | 1,2 2 | | |
| EDTA-Lösung | 1 | | 1 | | |
| wasserlöslicher Farbstoff | | | | 0,5 | |
| Alkohol | | | 10 | 15 | |
| Parfum | 0,4 | | | | |
| Wasser | ad 100 | ad 100 | ad 100 | ad 100 | ad 100 |

## Patentansprüche

1. Dünnflüssige Pickering-Emulsionen, welche feindisperse Systeme vom Typ Öl-in-Wasser darstellen und eine Viskosität von weniger als 3.000 mPas aufweisen, enthaltend
(1) eine Ölphase,
(2) eine Wasserphase,
(3) mindestens einen Typ mikrofeiner, anorganischer Partikel gewählt aus der Gruppe der Metalloxide, die
a) eine mittlere Partikelgröße von weniger als 200 nm haben,
b) sowohl hydrophile als auch lipophile Eigenschaften zeigen,
c) sowohl in lipophilen als auch in hydrophilen Flüssigkeiten dispergierbar sind und
d) gegebenenfalls oberflächlich beschichtet sind,
(4) 0,01 bis 5 Gew.-% mindestens eines polymeren nichtionischen und/oder kationischen Verdickungsmittels gewählt aus der Gruppe Hydroxyethylcellulose und Polyquaternium 37 und
(5) weniger als 0,5 Gew.-% eines oder mehrerer Emulgatoren.

2. Pickering-Emulsionen nach Anspruch 1, **dadurch gekennzeichnet, daß** sie emulgatorfrei sind.

3. Pickering-Emulsionen nach einem der vorhergehenden Ansprüche, **dadurch gekennzeichnet, daß** der Gehalt der verwendeten Partikel zwischen 1 Gew.-% und 15 Gew.-% ist, bezogen auf das Gesamtgewicht der Zubereitungen.

4. Pickering-Emulsionen nach einem der vorhergehenden Ansprüche,' **dadurch gekennzeichnet, daß** der Partikeldurchmesser der verwendeten Partikel zwischen 5 nm und 100 nm liegt.

5. Pickering-Emulsionen nach einem der vorhergehenden Ansprüche, **dadurch gekennzeichnet, daß** als polymeres Verdickungsmittel Hydroxyethylcellulose gewählt wird.

6. Pickering-Emulsionen nach einem der vorhergehenden Ansprüche, **dadurch gekennzeichnet, daß** als polymeres Verdickungsmittel Polyquaternium 37 gewählt wird.

7. Pickering-Emulsionen nach einem der vorhergehenden Ansprüche, **dadurch gekennzeichnet, daß** die Gesamtmenge an einem oder mehreren polymeren Verdickungsmitteln in den fertigen kosmetischen oder dermatologischen Zubereitungen kleiner als 5,0 Gew.-%, bevorzugt zwischen 0,05 und 3 Gew.-% gewählt wird, jeweils bezogen auf das Gesamtgewicht der Zubereitungen.

8. Pickering-Emulsionen nach einem der vorhergehenden Ansprüche, **dadurch gekennzeichnet, daß** sie eine oder mehrere UV-Filtersubstanzen enthalten.

9. Kosmetische und dermatologische Tücher, wobei die Tücher aus einem wasserunlöslichen Vlies bestehen, welches mit einer kosmetischen oder dermatologischen Tränkungslösung befeuchtet ist, die eine Pickering-Emulsion nach einem der vorhergehenden Ansprüche darstellt, wobei die Viskosität der Pickering-Emulsion kleiner als 2.000 mPas gewählt wird.

10. Tücher nach Anspruch 8, **dadurch gekennzeichnet, daß** das Gewichtsverhältnis des ungetränkten Tuchs zu der Tränkungslösung aus dem Bereich von 1 : 1 bis 1 : 5 gewählt wird.

11. Verfahren zur Herstellung von O/W-Pickering-Emulsionen mit einer Viskosität von weniger als 3.000 mPas, **dadurch gekennzeichnet, daß** eine O/W-Primäremulsion, welche
(1) eine Ölphase,
(2) eine Wasserphase,
(3) mindestens einen Typ mikrofeiner, anorganischer Partikel gewählt aus der Gruppe der Metalloxide, die
a) eine mittlere Partikelgröße von weniger als 200 nm haben,
b) sowohl hydrophile als auch lipophile Eigenschaften zeigen,
c) sowohl in hydrophilen als auch in lipophilen Medien dispergierbar sind,
enthält und welche eine Viskosität von 8.000 mPa·s oder mehr hat, mit einer bis zu zwanzigfachen Menge einer wässrigen Lösung, in der ein geeignetes Verdickungmittel gleichmäßig verteilt vorliegt, vermischt wird, wobei die O/W-Pickering-Emulsionen weniger als 0,5 Gew.-% eines oder mehrerer Emulgatoren enthalten.

## Claims

1. Low-viscosity Pickering emulsions which represent finely disperse systems of the oil-in-water type and have a viscosity of less than 3000 mPa·s, comprising
(1) an oil phase,
(2) a water phase,
(3) at least one type of microfine, inorganic particle chosen from the group of metal oxides which
a) have an average particle size of less than 200 nm,
b) exhibit both hydrophilic and also lipophilic properties,
c) are dispersible both in lipophilic and also in hydrophilic liquids and
d) are optionally surface-coated,
(4) 0.01 to 5% by weight of at least one polymeric nonionic and/or cationic thickener chosen from the group consisting of hydroxyethylcellulose and polyquaternium 37 and
(5) less than 0.5% by weight of one or more emulsifiers.

2. Pickering emulsions according to Claim 1, **characterized in that** they are emulsifier-free.

3. Pickering emulsions according to any of the preceding claims, **characterized in that** the content of the particles used is between 1 % by weight and 15% by weight, based on the total weight of the preparations.

4. Pickering emulsions according to any of the preceding claims, **characterized in that** the particle diameter of the particles used is between 5 nm and 100 nm.

5. Pickering emulsions according to any of the preceding claims, **characterized in that** hydroxyethylcellulose is chosen as polymeric thickener.

6. Pickering emulsions according to any of the preceding claims, **characterized in that** polyquaternium 37 is chosen as polymeric thickener.

7. Pickering emulsions according to any of the preceding claims, **characterized in that** the total amount of one or more polymeric thickeners in the finished cosmetic or dermatological preparations is chosen to be less than 5.0% by weight, preferably between 0.05 and 3% by weight, in each case based on the total weight of the preparations.

8. Pickering emulsions according to any of the preceding claims, **characterized in that** they comprise one or more UV filter substances.

9. Cosmetic and dermatological wipes, where the wipes consist of a water-insoluble nonwoven which is moistened with a cosmetic or dermatological impregnation solution which represents a Pickering emulsion according to one of the preceding claims, where the viscosity of the Pickering emulsion is chosen to be less than 2000 mPa·s.

10. Wipes according to Claim 8, **characterized in that** the weight ratio of the unimpregnated wipe to the impregnation solution is chosen from the range from 1:1 to 1:5.

11. Method of preparing O/W Pickering emulsions with a viscosity of less than 3000 mPa·s, **characterized in that** an O/W primary emulsion which comprises
(1) an oil phase,
(2) a water phase,
(3) at least one type of microfine inorganic particle chosen from the group of metal oxides which
a) have an average particle size of less than 200 nm,
b) exhibit both hydrophilic and also lipophilic properties,
c) are dispersible both in hydrophilic and also in lipophilic media,
and which has a viscosity of 8000 mPa·s or more is mixed with an up to twenty-fold amount of an aqueous solution in which a suitable thickener is present in an evenly distributed form, where O/W Pickering emulsions comprise less than 0.5% by weight of one or more emulsifiers.

## Revendications

1. Emulsions Pickering peu visqueuses, qui constituent des systèmes finement dispersés du type huile-dans-eau et qui présentent une viscosité de moins de 3 000 mPas, contenant
(1) une phase huileuse,
(2) une phase aqueuse,
(3) au moins un type de particule inorganique microfine, sélectionné parmi le groupe des oxydes métalliques,
a) qui ont une grandeur de particule moyenne de moins de 200 nm,
b) qui manifestent des propriétés non seulement hydrophiles mais aussi lipophiles,
c) qui peuvent être dispersés dans des liquides non seulement lipophiles mais aussi hydrophiles,
d) qui sont, le cas échéant, munis d'un revêtement en surface,
(4) de 0,01 à 5% en poids d'au moins un agent épaississant polymère, cationique et/ou non ionique, sélectionné parmi le groupe de l'hydroxyéthylcellulose et du polyquaternium 37 et
(5) de moins de 0,5% en poids d'un ou de plusieurs émulsifiants.

2. Émulsions Pickering selon la revendication 1, **caractérisées en ce qu'**elles sont exemptes d'émulsifiants.

3. Émulsions Pickering selon l'une quelconque des revendications précédentes, **caractérisées en ce que** la teneur des particules utilisées est comprise entre 1 % en poids et 15% en poids, par rapport au poids total des préparations.

4. Émulsions Pickering selon l'une quelconque des revendications précédentes, **caractérisées en ce que** le diamètre de particule des particules utilisées se situe entre 5 nm et 100 nm.

5. Émulsions Pickering selon l'une quelconque des revendications précédentes, **caractérisées en ce que** l'on choisit, en tant qu'agent épaississant polymère, l'hydroxyéthylcellulose.

6. Émulsions Pickering selon l'une quelconque des revendications précédentes, **caractérisées en ce que** l'on choisit, en tant qu'agent épaississant polymère, le polyquaternium 37.

7. Émulsions Pickering selon l'une quelconque des revendications précédentes, **caractérisées en ce que** la quantité totale d'un ou de plusieurs agents épaississants polymères dans les préparations cosmétiques ou dermatologiques finies est choisie inférieure à 5,0 % en poids, de préférence, comprise entre 0,05 et 3 % en poids, à chaque fois, par rapport au poids total des préparations.

8. Émulsions Pickering selon l'une quelconque des revendications précédentes, **caractérisées en ce qu'**elles contiennent une ou plusieurs substances à filtre UV.

9. Étoffes cosmétiques ou dermatologiques, les étoffes se composant d'un non-tissé non hydrosoluble, qui est humidifié à l'aide d'une solution d'imprégnation cosmétique ou dermatologique, qui constitue une émulsion Pickering selon l'une quelconque des revendications précédentes, la viscosité de l'émulsion Pickering étant sélectionnée comme étant inférieure à 2 000 mPas.

10. Étoffes selon la revendication 8, **caractérisées en ce que** le rapport pondéral de l'étoffe non imprégnée à la solution d'imprégnation est choisi dans le domaine de 1:1 à 1:5.

11. Procédé en vue de la préparation d'émulsions Pickering H/E, ayant une viscosité de moins de 3 000 mPas, **caractérisé en ce qu'**une émulsion H/E primaire qui contient
(1) une phase huileuse,
(2) une phase aqueuse,
(3) au moins un type de particule inorganique micro fine, sélectionné parmi le groupe des oxydes métalliques,
a) qui ont une grandeur de particule moyenne de moins de 200 nm,
b) qui manifestent des propriétés non seulement hydrophiles mais aussi lipophiles,
c) qui peuvent être dispersés dans des milieux non seulement hydrophiles, mais aussi lipophiles,
et qui a une viscosité de 8 000 mPas ou plus, est mélangée avec une quantité qui est jusqu'à vingt fois plus grande d'une solution aqueuse, dans laquelle est présente, réparti d'une manière uniforme, un agent épaississant approprié, les émulsions Pickering H/E contenant moins de 0,5% en poids d'un ou de plusieurs émulsifiants.
